# EUROPEAN PATENT APPLICATION

(11) **EP 4 813 328 A1**
(43) Date of publication of application: **30.09.2026**
(21) Application number: 26166073.2
(22) Date of filing: 19.03.2026
(51) Int. Cl.: A61B 17/80, A61B 17/68, A61B 17/00

(54) **MEDICAL FIXATION DEVICE**

(30) Priority: 20.03.2025 KR 20250035802
(71) Applicant: Park, Hyung Joo, Seoul 05701 (KR); Oh, Gi Ae, Seoul 05701 (KR); Park, Kyu Won, Seoul 05701 (KR); Park, Kyumin, Seoul 05701 (KR)
(72) Inventor: PARK, Hyung Joo, 05701 Seoul (KR)
(74) Representative: EIP

(57) **Abstract**

A medical fixation device that is convenient to use during surgery and can stably fix body tissue is disclosed. The medical fixation device includes a fixation bar including a slot and a guide, a connecting bridge disposed to intersect with the fixation bar, and a coupler having a base coupled to the guide such that at least a portion thereof is received in the slot and having a connection head protruding from the base to couple with the connecting bridge.

## Description

### CROSS-REFERENCE TO RELATED APPLICATIONS

This application claims the priority of Korean Patent Application No. 10-2025-0035802 filed on March 20, 2025, in the Korean Intellectual Property Office, the disclosures of which are incorporated herein by reference.

### BACKGROUND

### Technical Field

The present invention relates to a medical fixation device, and more particularly, to a medical fixation device capable of fixing body tissues such as bones during surgery.

### Background Art

Generally, various types of medical fixation devices are used in various medical fields such as orthopedics and thoracic surgery to fix body tissues such as bones during surgery.

For example, in the case of pectus excavatum surgery, a type of chest deformity, a medical fixation device is essentially used to fix the depressed sternum in a normal position after lifting it. In such surgeries, since organs directly related to the patient's life are involved, quick and accurate surgery is required.

Conventional medical fixation devices had problems such as inconvenient use during surgery or insufficient strength to fix body tissues. Additionally, there were problems such as having to widen the incision area more than necessary to install the fixation device.

Research and development to improve medical fixation devices has been continuously carried out to solve these problems.

### SUMMARY

A feature to be achieved by the present disclosure is to provide a medical fixation device that is convenient to use during surgery and can stably fix body tissues.

A medical fixation device according to an embodiment of the present invention includes: a fixation bar including a slot and a guide; a connecting bridge disposed to intersect with the fixation bar; and a coupler having a base coupled to the guide such that at least a portion thereof is received in the slot and having a connection head protruding from the base to couple with the connecting bridge.

The slot may include an entrance having a size through which the base can pass and an extension part extending from the entrance in the longitudinal direction of the fixation bar. The base may be coupled to the guide in the extension part so that movement in the thickness direction of the fixation bar is restricted.

The guide may be disposed in the extension part, and an inclined surface may be disposed at the end of the guide adjacent to the entrance.

The base may include a base bottom in contact with one surface of the guide, a base top in contact with the other surface of the guide, and a groove disposed between the base bottom and the base top for the guide to be inserted.

The guide may be disposed in the slot as a pair facing each other.

The base may be capable of angle adjustment between the pair of guides to be coupled with or separated from the pair of guides.

At least one of the base bottom and the base top may have a width that can pass through a gap between the pair of guides.

Alternatively, one of the base bottom and the base top may have a width that can pass through the gap between the pair of guides, and the other may have a width that cannot pass through the gap.

The connecting bridge may include a through hole into which the connection head is inserted.

The medical fixation device may further include a fixing member coupled to the connection head to press the connecting bridge toward the base.

The connection head may have a thread on an outer circumferential surface thereof, and the fixing member may have an insertion hole with a thread that engages with the thread of the connection head.

The fixation bar may have a curved shape so as to be insertable into a human body and to fix a specific part of the human body.

The fixation bar, the connecting bridge, and the coupler may each be made of a biocompatible material.

The medical fixation device according to the present invention can be simply coupled by inserting the coupler into the slot of the fixation bar. Therefore, the coupler can be simply coupled to the fixation bar during surgery, and the fixing member can be smoothly coupled to the coupler.

In addition, since the coupler of the medical fixation device according to the present invention can move along the guide, the user can adjust the connection position of the connecting bridge in real-time according to the patient's condition or anatomical characteristics even during surgery. Therefore, the safety of the surgery is improved, the surgery time is shortened, and the risk of reoperation can be minimized.

The features of the present disclosure are not limited to the aforementioned features, and other objects, which are not mentioned above, will be understood by a person having ordinary skill in the art from the following description.

### BRIEF DESCRIPTION OF THE DRAWINGS

The above and other aspects, features and other characteristics of the present invention embodiments will be more clearly understood from the following detailed description taken in conjunction with the accompanying drawings, in which:
FIG. 1 is a perspective view showing a medical fixation device according to an embodiment of the present invention;
FIG. 2 is a perspective view showing a fixation bar of a medical fixation device according to an embodiment of the present invention;
FIG. 3 is a view showing an example of use of a fixation bar of a medical fixation device according to an embodiment of the present invention;
FIG. 4 is a front view showing a coupler of a medical fixation device according to an embodiment of the present invention;
FIG. 5 is a perspective view showing a coupler of a medical fixation device according to an embodiment of the present invention inserted into a slot of a fixation bar;
FIG. 6 is a perspective view showing a coupler of a medical fixation device according to an embodiment of the present invention coupled to a guide of a fixation bar;
FIG. 7 is an exploded perspective view showing a part of a medical fixation device according to an embodiment of the present invention;
FIG. 8 is a cross-sectional view showing a part of a medical fixation device according to an embodiment of the present invention;
FIG. 9 shows a modified example of the arrangement form of a fixation bar of a medical fixation device according to an embodiment of the present invention;
FIG. 10 is a perspective view showing a part of a medical fixation device according to another embodiment of the present invention;
FIG. 11 is a cross-sectional view showing a part of a medical fixation device according to yet another embodiment of the present invention;
FIG. 12 is a plan view showing a coupler of the medical fixation device shown in FIG. 11 inserted into a slot of a fixation bar;
FIG. 13 is a cross-sectional view showing a coupler of the medical fixation device shown in FIG. 11 inserted into a slot of a fixation bar;
FIG. 14 is a plan view showing a coupler of the medical fixation device shown in FIG. 11 coupled to a guide of a fixation bar;
FIG. 15 is a cross-sectional view showing a coupler of the medical fixation device shown in FIG. 11 coupled to a guide of a fixation bar;
FIG. 16 is a cross-sectional view showing a part of a medical fixation device according to yet another embodiment of the present invention;
FIG. 17 is a cross-sectional view showing a coupler of the medical fixation device shown in FIG. 16 coupled to a guide of a fixation bar;
FIG. 18 is a cross-sectional view showing a part of a medical fixation device according to yet another embodiment of the present invention;
FIG. 19 is a cross-sectional view showing a coupler of the medical fixation device shown in FIG. 18 coupled to a guide of a fixation bar; and
FIG. 20 is a view showing an example of prior art.

### DETAILED DESCRIPTION OF THE EMBODIMENT(S)

The advantages and features of this specification, and methods for achieving them will become apparent by reference to embodiments described below in conjunction with the accompanying drawings. However, this specification is not limited to the embodiments disclosed herein but will be implemented in various different forms, and these embodiments are provided only to make the disclosure of this specification complete and to fully convey the scope of the specification to those skilled in the art to which this specification belongs.

The shapes, areas, ratios, angles, numbers, etc. disclosed in the drawings for describing embodiments of this specification are exemplary, and this specification is not limited to the illustrated matters. Throughout the specification, the same reference numbers refer to the same components. Also, in explaining this specification, detailed descriptions of related known technologies may be omitted if they are deemed to unnecessarily obscure the gist of this specification. When terms such as 'include', 'have', 'consist of, etc. are used in this specification, other parts may be added unless 'only' is used. When a component is expressed in the singular, it includes the plural case unless there is a specific explicit statement.

In interpreting components, it is interpreted to include an error range even without a separate explicit statement.

In the case of descriptions of positional relationships, for example, when the positional relationship between two parts is described as 'on', 'above', 'below', 'beside', etc., one or more other parts may be positioned between the two parts unless 'directly' is used.

Also, first, second, etc. are used to describe various components, but these components are not limited by these terms. These terms are used only to distinguish one component from another component.

The same reference numbers throughout the drawings refer to the same components.

The area and thickness of each structure shown in the drawings are illustrated for convenience of explanation, and this specification is not necessarily limited to the area and thickness of the illustrated structure.

In the embodiments, a 'module' or 'unit' performs at least one function or operation and may be implemented in hardware or software, or a combination of hardware and software. Also, a plurality of 'modules' or a plurality of 'units' may be integrated into at least one module except for 'modules' or 'units' that need to be implemented in specific hardware.

Each feature of the various embodiments of this specification may be partially or wholly combined or combined with each other, various interlocking and driving are technically possible, and the embodiments may be implemented independently of each other or may be implemented together in an associated relationship.

Hereinafter, the present invention will be described with reference to the drawings.

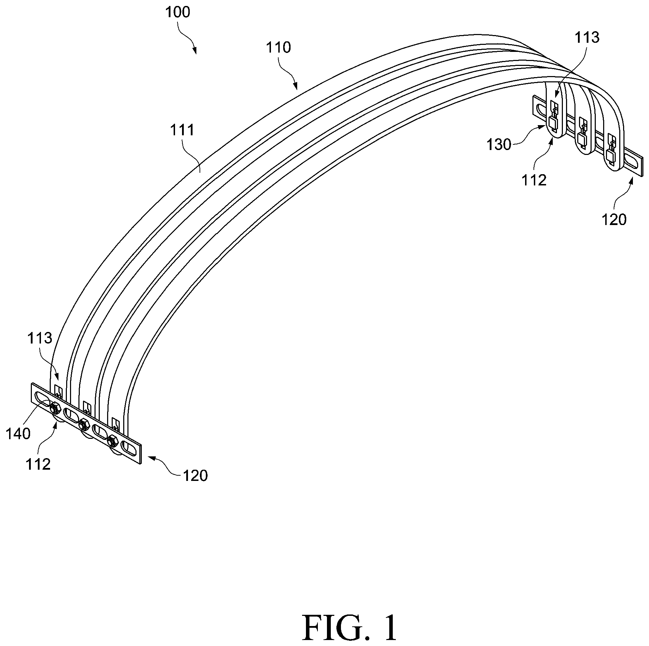

FIG. 1 is a perspective view showing a medical fixation device according to an embodiment of the present invention.

As shown in the drawing, the medical fixation device 100 according to an embodiment of the present invention includes a bar 110 for fixing body tissue, a connecting bridge 120 connected to the bar 110, a fastening member 130 connecting the bar 110 and the connecting bridge 120, and a fixing member 140 coupled to the fastening member 130. Hereinafter, the bar is referred to as a fixation bar, and the fastening member is referred to as a coupler.

The medical fixation device 100 according to an embodiment of the present invention can be used to fix a specific part of the body or body tissue such as bones in various medical fields such as orthopedics and thoracic surgery.

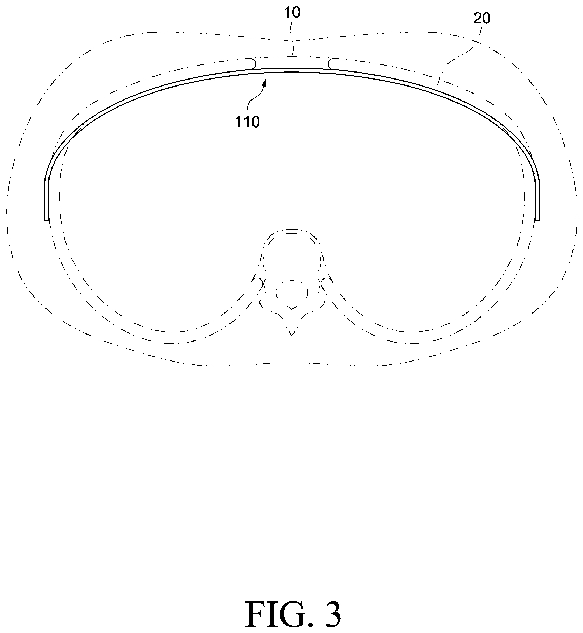

Hereinafter, as an example, the medical fixation device 100 according to an embodiment of the present invention is described as being used in pectus excavatum correction surgery. During pectus excavatum correction surgery, as shown in FIG. 3, the patient's sternum 10 can be lifted and fixed in the lifted state by inserting a fixation bar 110 into the patient's chest. The fixation bar 110 inserted into the patient's chest can stably support the sternum 10 by being fixed to the ribs 20.

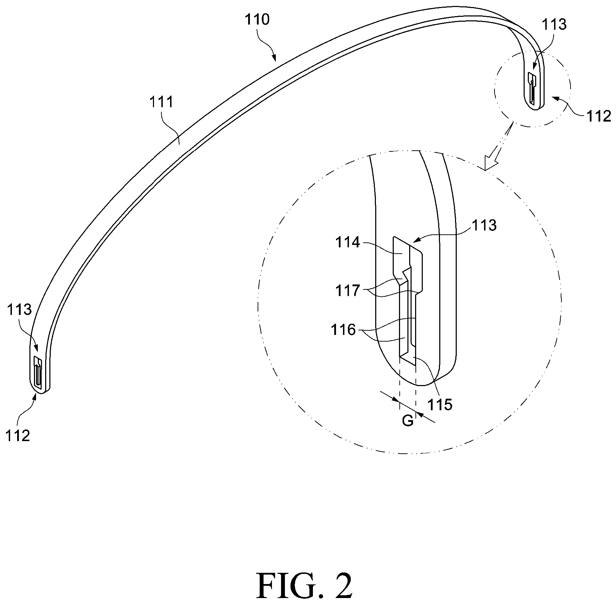

Referring to FIGS. 1 to 3, the fixation bar 110 includes a body 111 for fixing or supporting body tissue. The body 111 includes a pair of ends 112 to which the connecting bridge 120 is connected. A slot 113 is disposed at each end 112.

The body 111 may be made of a biocompatible material that is excellent in corrosion resistance, has high durability, and is harmless to the human body. Also, the body 111 may be curved to support the sternum 10 in a lifted state. Furthermore, since the body 111 must support the sternum 10 in a lifted state, it has sufficient strength and elasticity.

The slot 113 is arranged to penetrate the body 111 in the thickness direction. A pair of guides 116 are arranged facing each other inside the slot 113. The slot 113 includes an entrance 114 and an extension part 115 arranged sequentially in the longitudinal direction of the body 111. The entrance 114 has a relatively wide width so that the coupler 130 can completely pass through. The extension part 115 extends from the entrance 114 to ensure a moving distance for the coupler 130 to move in the longitudinal direction of the fixation bar 110 within the slot 113.

By entering the coupler 130 through the entrance 114 and moving it to the extension part 115, the coupler 130 can be coupled with the guide 116. The guide 116, by coupling with the coupler 130, can prevent the coupler 130 from escaping in at least one direction. Specifically, the guide 116 restricts the movement of the coupler 130 so that it cannot escape from the fixation bar 110 in the thickness direction of the fixation bar 110. Therefore, the coupler 130 cannot be separated from the fixation bar 110 in the extension part 115 but can move in the longitudinal direction of the fixation bar 110.

The pair of guides 116 are arranged to face each other within the extension part 115 and extend along the extension part 115.

The guide 116 can be arranged inside the slot 113 so as not to protrude from the body 111. Since the guide 116 does not protrude from the surface of the body 111, the surface of the body 111 can be maintained smooth. Therefore, when the fixation bar 110 is inserted into the human body, it can prevent the problem of the guide 116 irritating or damaging body tissue.

Also, one surface of the guide 116 can be arranged on the same plane as one surface of the body 111. By placing one surface of the guide 116 on the same plane as one surface of the body 111, the process of precision machining the guide 116 is simplified, and the manufacture of the fixation bar 110 can be made easier.

An inclined surface 117 is arranged at one end of the guide 116, specifically at the end of the guide 116 adjacent to the entrance 114. By arranging the inclined surface 117 at the end of the guide 116, when the coupler 130 moves from the entrance 114 to the extension part 115, the guide 116 can be coupled with the coupler 130 more smoothly.

The specific configuration of the fixation bar 110 is not limited to what is shown. For example, the shape or size of the body 111 can be variously changed according to the patient's body type or the characteristics of the body tissue to be fixed. Also, the number, shape, and position of the slot 113 can be variously changed. In addition, the number, shape, and position of the guide 116 can also be variously changed.

The drawings show three fixation bars 110 connected at regular intervals by the connecting bridge 120, but the fixation bars 110 can be inserted into the human body to fix body tissue in various numbers.

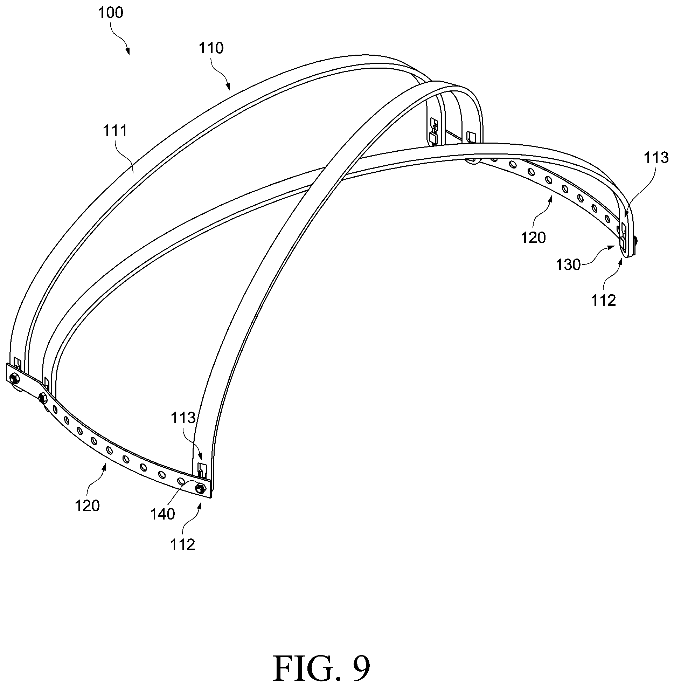

Also, as shown in FIG. 9, when the medical fixation device 100 is used for pectus excavatum correction surgery, two of the three fixation bars 110 can be connected to the connecting bridge 120 so that they intersect with each other.

Also, although the drawings show two guides 116 arranged in the slot 113, the number or position of the guides 116 can be variously changed.

The connecting bridge 120 is configured to connect multiple fixation bars 110 to each other. The connecting bridge 120 is arranged to intersect with the fixation bar 110 and can connect multiple fixation bars 110. The connecting bridge 120 can be coupled to each of both ends 112 of the fixation bar 110.

The connecting bridge 120 has multiple through holes 121. The multiple through holes 121 can be arranged at regular intervals along the longitudinal direction of the connecting bridge 120. The coupler 130 is partially inserted into the through hole 121.

The connecting bridge 120, like the fixation bar 110, can be made of a biocompatible material that is excellent in corrosion resistance, has high durability, and is harmless to the human body. Also, the connecting bridge 120 has sufficient strength and durability to function stably in the human body for a long period after surgery.

The specific configuration of the connecting bridge 120 is not limited to what is shown and can be variously changed. For example, the length or width of the connecting bridge 120 can be variously manufactured according to the number or position of the fixation bars 110 to be fixed, and the number or arrangement interval of the through holes 121 can also be changed as needed.

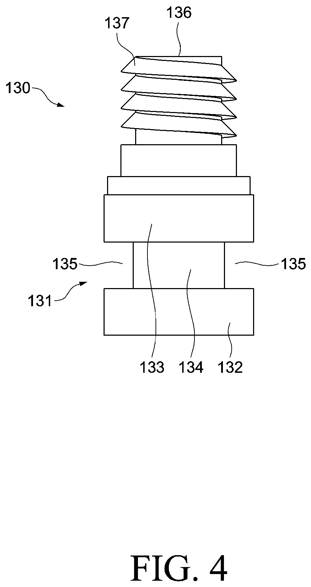

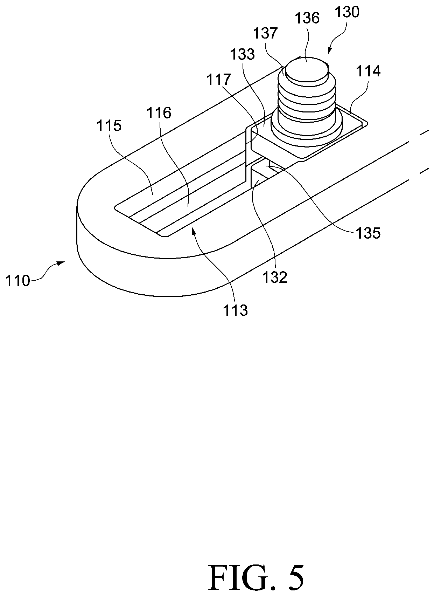

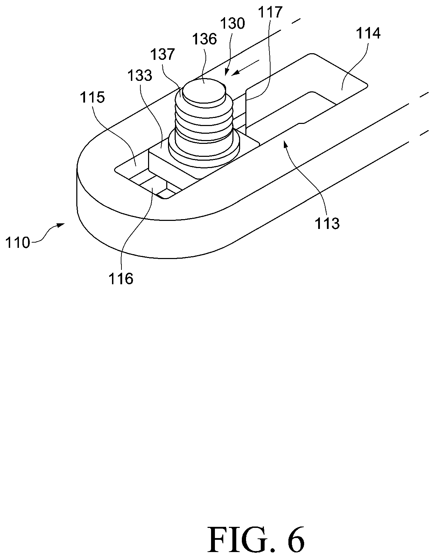

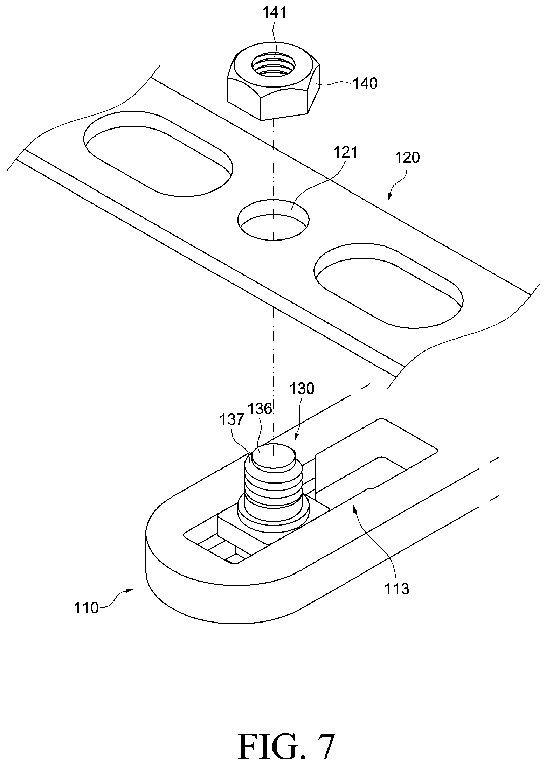

Referring to FIGS. 1, 4 to 7, the coupler 130 connects the fixation bar 110 and the connecting bridge 120, and combines them along with the fixing member 140. The coupler 130 can be coupled with the fixation bar 110 by inserting it into the slot 113 and moving it. The coupler 130 is coupled with the guide 116 in the extension part 115 of the slot 113 and does not separate from the fixation bar 110. Therefore, during surgery, the user does not have to separately support the coupler 130 by hand, and can easily couple the connecting bridge 120 or the fixing member 140 to the coupler 130.

The coupler 130 includes a base 131 and a connection head 136.

The base 131 is coupled to the guide 116. The base 131 includes a base bottom 132 in contact with one surface of the guide 116, a base top 133 in contact with the other surface of the guide 116, and a base middle 134 connecting the base bottom 132 and the base top 133. The base bottom 132 and the base top 133 have a width that cannot pass through the gap G between the pair of guides 116. Therefore, the base 131 cannot escape in one direction in the extension part 115. That is, the movement of the base 131 in the thickness direction of the fixation bar 110 is restricted in the extension part 115.

A pair of grooves 135 are arranged between the base bottom 132 and the base top 133. The base 131 is coupled with the fixation bar 110 in such a way that the pair of guides 116 are each inserted into the pair of grooves 135. The base 131 can move along the guide 116.

The connection head 136 protrudes from the base 131 so that it can be connected to the connecting bridge 120. The connection head 136 can pass through the through hole 121 of the connecting bridge 120. A thread 137 is arranged on the outer circumferential surface of the connection head 136, which engages with the fixing member 140.

The coupler 130 can be made of a biocompatible material that may be excellent in corrosion resistance, have high durability, and may be harmless to the human body. Also, the coupler 130 may have sufficient strength and durability to function stably in the human body for a long period after surgery.

The specific configuration of the coupler 130 is not limited to what is shown and can be variously changed. For example, the shape or size of the base 131 can be variously changed according to the shape or size of the guide 116.

The fixing member 140, along with the coupler 130, combines the fixation bar 110 and the connecting bridge 120. The fixing member 140 includes an insertion hole 141 into which the connection head 136 is inserted. Inside the insertion hole 141, a thread is arranged that engages with the thread 137 of the connection head 136.

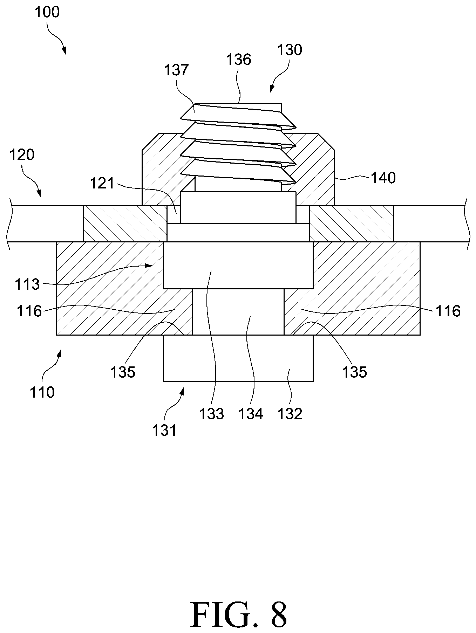

As shown in FIG. 8, when the fixing member 140 is fastened to the connection head 136, the fixing member 140 contacts the connecting bridge 120, pushing the connecting bridge 120 toward the fixation bar 110. Therefore, the connecting bridge 120 can be firmly fixed to the fixation bar 110.

The fixing member 140 can be made of a biocompatible material that is excellent in corrosion resistance, has high durability, and is harmless to the human body. Also, the fixing member 140 has sufficient strength and durability to function stably in the human body for a long period after surgery.

Also, although the drawings show the fixing member 140 having a nut structure that engages with the coupler 130, the fixing member 140 can be changed into various other structures that are coupled with the coupler 130 to fix the connecting bridge 120 to the fixation bar 110.

As described above, the medical fixation device 100 according to an embodiment of the present invention can be simply coupled to the fixation bar 110 by inserting the coupler 130 into the slot 113 of the fixation bar 110. Therefore, it is easy to couple the fixing member 140 to the coupler 130 during surgery, and the connecting bridge 120 can be fixed more smoothly.

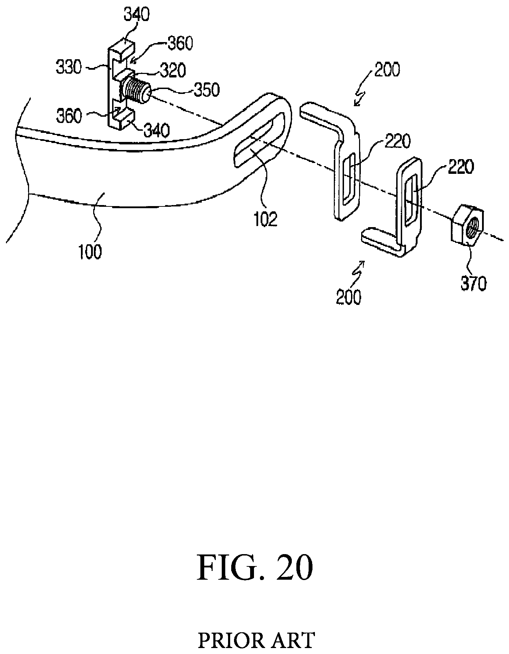

In the case of the prior art (U.S. Patent No. 9833269) shown in FIG. 20, to fix the rib fixing part 200 to the correction bar 100, the user has to use a separate tool to insert a fixing screw unit including a screw 350 into the through hole 102 of the correction bar 100 without moving it, and then fasten the fixing member 370. Therefore, there are problems such as the fastening of the fixing member 370 to the fixing screw unit during surgery being very cumbersome, difficulty in fixing the fixing screw unit at a predetermined position on the correction bar 100, and delays in surgery time.

In contrast, the medical fixation device 100 according to an embodiment of the present invention can be easily coupled to the fixation bar 110 during surgery by inserting the coupler 130 into the slot 113 of the fixation bar 110 and moving it, and since the coupler 130 is fixed to the fixation bar 110, fastening of the fixing member 140 is easy. Therefore, it has the effect of being convenient to use and can shorten surgery time.

In addition, since the coupler 130 of the medical fixation device 100 according to an embodiment of the present invention can move along the guide 116 while maintaining the coupled state with the fixation bar 110, the user can easily adjust the fixing position according to the patient's condition or anatomical characteristics even during surgery. Therefore, the safety of the surgery is improved, the surgery time is shortened, and the risk of reoperation can be minimized.

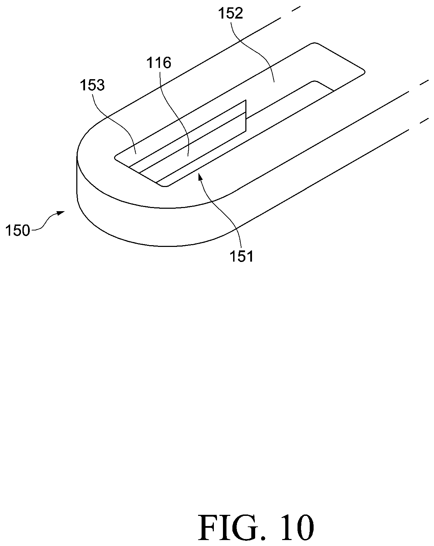

FIG. 10 is a perspective view showing a part of a medical fixation device according to another embodiment of the present invention.

The fixation bar 150 of the medical fixation device shown in FIG. 10 includes a slot 151 into which a part of the coupler 130 can be inserted. The slot 151 includes an entrance 152 and an extension part 153. A guide 116 to which the coupler 130 is coupled is arranged in the extension part 153. No guide is arranged in the entrance 152. The width of the entrance 152 and the width of the extension part 153 can be the same. The coupler 130 can enter through the entrance 152, and by moving from the entrance 152 to the extension part 153, it can be coupled to the guide 116.

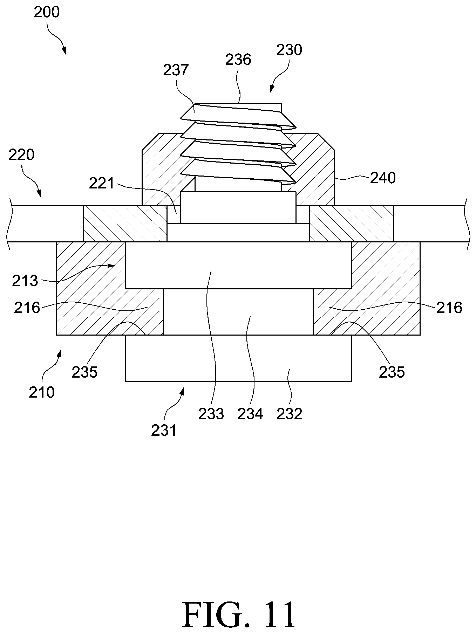

FIG. 11 is a cross-sectional view showing a part of a medical fixation device according to yet another embodiment of the present invention.

As shown in FIG. 11, the medical fixation device 200 according to yet another embodiment of the present invention includes a fixation bar 210 for fixing body tissue, a connecting bridge 220 connected to the fixation bar 210, a coupler 230 for fixing the connecting bridge 220 to the fixation bar 210, and a fixing member 240.

The fixation bar 210 includes a slot 213 into which the coupler 230 is inserted. The slot 213 includes an entrance 214 and an extension part 215. A pair of guides 216 to which the coupler 230 is coupled are arranged in the extension part 215. No guide is arranged in the entrance 214. The width of the entrance 214 and the width of the extension part 215 can be the same. The coupler 230 can enter through the entrance 214.

The pair of guides 216 are arranged to face each other within the extension part 215 and extend in the longitudinal direction of the fixation bar 210. An inclined surface 217 is arranged at the end of the guide 216 adjacent to the entrance 214. By arranging the inclined surface 217 at the end of the guide 216, the coupler 230 can move more smoothly from the entrance 214 to the extension part 215.

The connecting bridge 220 is arranged to intersect with the fixation bar 210 and can connect multiple fixation bars 210. The connecting bridge 220 includes a through hole 221 into which a part of the coupler 230 is inserted.

The coupler 230 connects the fixation bar 210 and the connecting bridge 220, and combines them along with the fixing member 240. The coupler 230 includes a base 231 and a connection head 236.

The base 231 is movably coupled to the guide 216. The base 231 includes a base bottom 232 in contact with one surface of the guide 216, a base top 233 in contact with the other surface of the guide 216, and a base middle 234 connecting the base bottom 232 and the base top 233.

A pair of grooves 235 are arranged between the base bottom 232 and the base top 233. The base 231 is coupled with the fixation bar 210 in such a way that the pair of guides 216 are each inserted into the pair of grooves 235. The base 231 can move along the guide 216.

The base 231 is coupled with the pair of guides 216 in a way that allows angle adjustment between the pair of guides 216. That is, the base 231 is configured to either pass through the gap G between the pair of guides 216 or not pass through the gap G depending on the angle of the base relative to the guide 216. Specifically, the base bottom 232 and the base top 233 can have a rectangular shape with different side lengths.

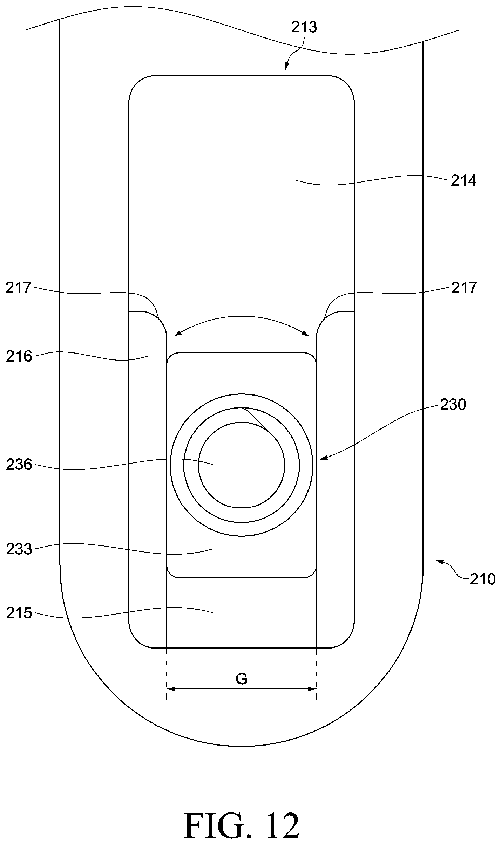

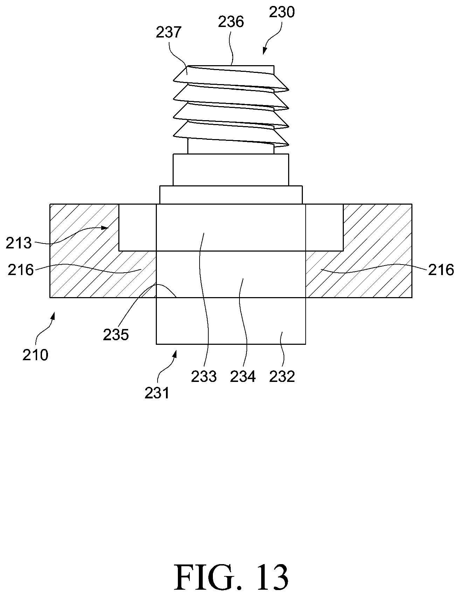

As shown in FIGS. 12 and 13, when the longer sides of the base bottom 232 and the base top 233 are placed parallel to the guide 216, the base 231 can pass through the gap G between the pair of guides 216, and the base 231 can be inserted into the slot 213. Also, the base 231 can rotate inside the slot 213.

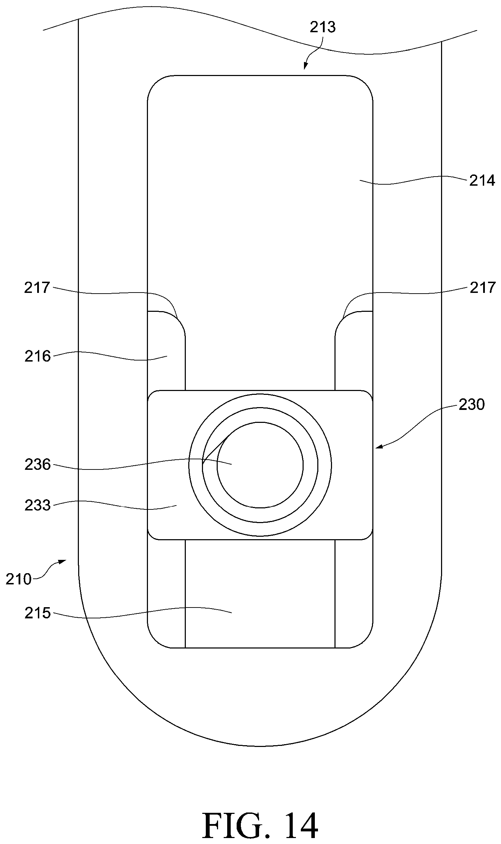

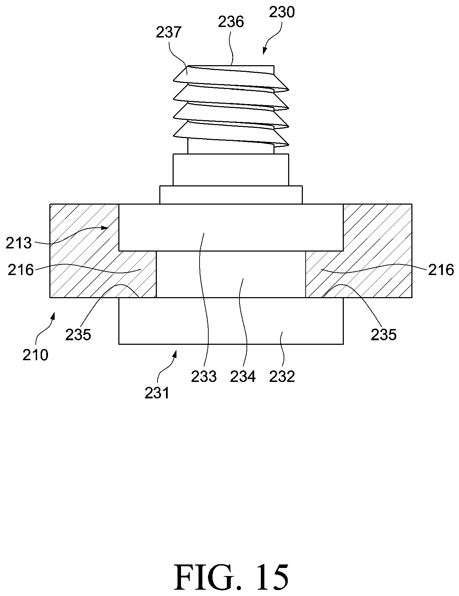

As shown in FIGS. 14 and 15, when the base 231 is rotated 90 degrees inside the slot 213, the longer sides of the base bottom 232 and the base top 233 are placed perpendicular to the guide 216. At this time, the guide 216 is inserted into the groove 235 of the base 231, the base bottom 232 contacts one surface of the guide 216, and the base top 233 contacts the other surface of the guide 216. Therefore, the movement of the base 231 in the thickness direction of the fixation bar 210 is restricted inside the slot 213.

The connection head 236 protrudes from the base 231 so that it can be connected to the connecting bridge 220. The connection head 236 can pass through the through hole 221 of the connecting bridge 220. A thread 237 is arranged on the outer circumferential surface of the connection head 236, which engages with the fixing member 240.

The fixing member 240, along with the coupler 230, combines the fixation bar 210 and the connecting bridge 220. The fixing member 240 includes an insertion hole into which the connection head 236 is inserted. Inside the insertion hole, a thread is arranged that engages with the thread 237 of the connection head 236.

When the fixing member 240 is fastened to the connection head 236, the fixing member 240 contacts the connecting bridge 220, pushing the connecting bridge 220 toward the fixation bar 210. Therefore, the connecting bridge 220 can be firmly fixed to the fixation bar 210.

The medical fixation device 200 according to yet another embodiment of the present invention has the coupler 230 coupled with the pair of guides 216 in a way that allows angle adjustment inside the slot 213. Therefore, the coupler 230 can be coupled to the fixation bar 210 or separated from the fixation bar 210 simply by rotating it inside the slot 213. Therefore, the coupling or separation of the coupler 230 is more convenient during surgery, and if necessary, only the coupler 230 can be quickly replaced.

Although the drawings show the guide 216 arranged only in a part of the slot 213, the guide 216 can be arranged throughout the entire slot 213. For example, if the base 231 of the coupler 230 is configured to pass through the gap G between the pair of guides 216, the slot 213 does not need to include a separate entrance for the entry and exit of the base 231, and the guide 216 can be arranged throughout the entire slot 213.

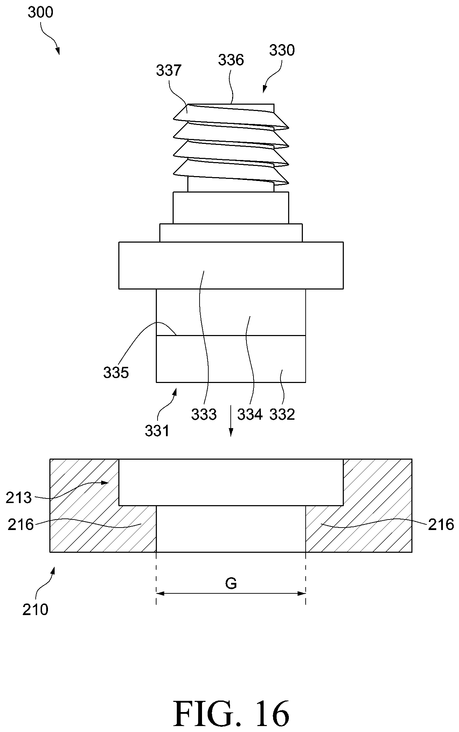

FIG. 16 is a cross-sectional view showing a part of a medical fixation device according to yet another embodiment of the present invention.

The medical fixation device 300 according to yet another embodiment of the present invention includes, in addition to the fixation bar 210 for fixing body tissue and the coupler 330 coupled to the fixation bar 210, the connecting bridge 220 and the fixing member 240 as described earlier.

The fixation bar 210 includes a slot 213 into which the coupler 330 is inserted, and a pair of guides 216 are arranged inside the slot 213. The specific configuration of the fixation bar 210 is as described earlier.

The coupler 330 connects the fixation bar 210 and the connecting bridge 220, and combines them along with the fixing member 240. The coupler 330 includes a base 331 and a connection head 336.

The base 331 is movably coupled to the guide 216. The base 331 includes a base bottom 332 in contact with one surface of the guide 216, a base top 333 in contact with the other surface of the guide 216, and a base middle 334 connecting the base bottom 332 and the base top 333.

A pair of grooves 335 are arranged between the base bottom 332 and the base top 333. The base 331 is coupled with the fixation bar 210 in such a way that the pair of guides 216 are each inserted into the pair of grooves 335. The base 331 can move along the guide 216.

The base 331 is coupled with the pair of guides 216 in a way that allows angle adjustment between the pair of guides 216. That is, the base 331 is configured to either pass through the gap G between the pair of guides 216 or not pass through the gap G depending on the angle of the base 331 relative to the guide 216. Specifically, the base bottom 332 is configured to pass through the gap G, and the base top 333 cannot pass through the gap G. The base bottom 332 can have a rectangular shape with different side lengths. The base top 333 can have a circular or polygonal shape of a size that cannot pass through the gap G.

As shown in FIG. 16, when the longer side of the base bottom 332 is placed parallel to the guide 216, the base bottom 332 can pass through the gap G between the pair of guides 216, and the base 331 can be inserted into the slot 213. The base 331 can rotate inside the slot 213.

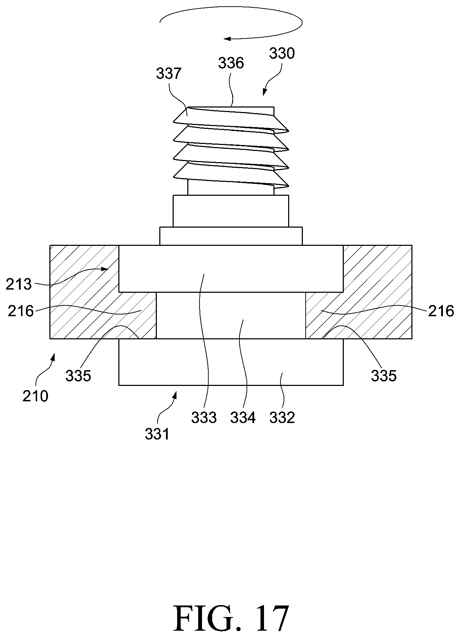

As shown in FIG. 17, when the base 331 is rotated 90 degrees inside the slot 213, the longer side of the base bottom 332 is placed perpendicular to the guide 216. At this time, the guide 216 is inserted into the groove 335 of the base 331, the base bottom 332 contacts one surface of the guide 216, and the base top 333 contacts the other surface of the guide 216. Therefore, the movement of the base 331 in the thickness direction of the fixation bar 210 is restricted inside the slot 213. If necessary, the size and shape of the base 331 can be variously modified, and the base 331 can be configured to move in the longitudinal direction of the fixation bar 210 inside the slot 213.

The connection head 336 protrudes from the base 331 so that it can be connected to the connecting bridge 220. The connection head 336 can pass through the through hole 221 of the connecting bridge 220. A thread 337 is arranged on the outer circumferential surface of the connection head 336, which engages with the fixing member 240.

The medical fixation device 300 according to yet another embodiment of the present invention has the coupler 330 coupled with the pair of guides 216 by rotating inside the slot 213, so the coupler 330 can be separated from the fixation bar 210 simply by rotating it inside the slot 213.

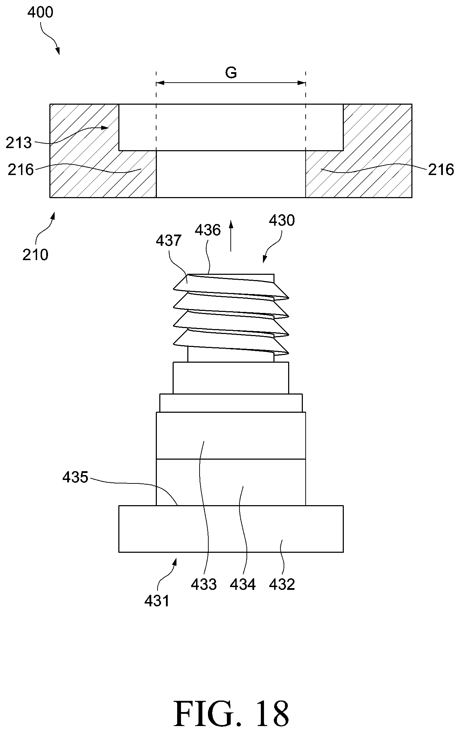

FIG. 18 is a cross-sectional view showing a part of a medical fixation device according to yet another embodiment of the present invention.

The medical fixation device 400 according to yet another embodiment of the present invention includes, in addition to the fixation bar 210 for fixing body tissue and the coupler 430 coupled to the fixation bar 210, the connecting bridge 220 and the fixing member 240 as described earlier.

The fixation bar 210 includes a slot 213 into which the coupler 430 is inserted, and a pair of guides 216 are arranged inside the slot 213. The specific configuration of the fixation bar 210 is as described earlier.

The coupler 430 connects the fixation bar 210 and the connecting bridge 220, and combines them along with the fixing member 240. The coupler 430 includes a base 431 and a connection head 436.

The base 431 is movably coupled to the guide 216. The base 431 includes a base bottom 432 in contact with one surface of the guide 216, a base top 433 in contact with the other surface of the guide 216, and a base middle 434 connecting the base bottom 432 and the base top 433.

A pair of grooves 435 are arranged between the base bottom 432 and the base top 433. The base 431 is coupled with the fixation bar 210 in such a way that the pair of guides 216 are each inserted into the pair of grooves 435. The base 431 can move along the guide 216.

The base 431 is coupled with the pair of guides 216 in a way that allows angle adjustment between the pair of guides 216. That is, the base 431 is configured to either pass through the gap G between the pair of guides 216 or not pass through the gap G depending on the angle of the base 431 relative to the guide 216. Specifically, the base top 433 is configured to pass through the gap G, and the base bottom 432 cannot pass through the gap G. The base top 433 can have a rectangular shape with different side lengths. The base bottom 432 can have a circular or polygonal shape of a size that cannot pass through the gap G.

As shown in FIG. 18, when the longer side of the base top 433 is placed parallel to the guide 216, the base top 433 can pass through the gap G between the pair of guides 216, and the base 431 can be inserted into the slot 213. The base 431 can rotate inside the slot 213.

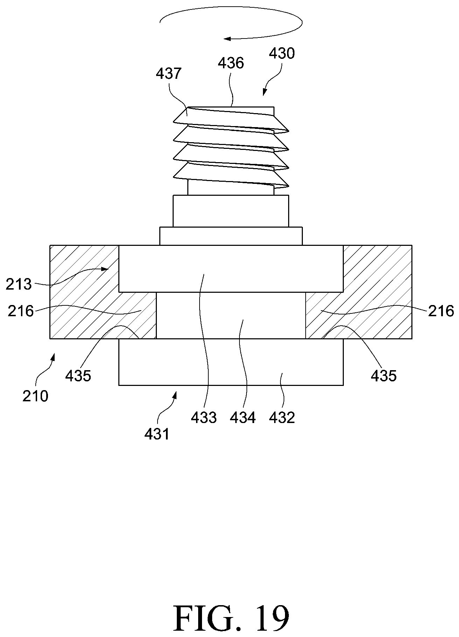

As shown in FIG. 19, when the base 431 is rotated 90 degrees inside the slot 213, the longer side of the base top 433 is placed perpendicular to the guide 216. At this time, the guide 216 is inserted into the groove 435 of the base 431, the base bottom 432 contacts one surface of the guide 216, and the base top 433 contacts the other surface of the guide 216. Therefore, the movement of the base 431 in the thickness direction of the fixation bar 210 is restricted inside the slot 213. If necessary, the base 431 can move in the longitudinal direction of the fixation bar 210 inside the slot 213.

The connection head 436 protrudes from the base 431 so that it can be connected to the connecting bridge 220. The connection head 436 can pass through the through hole 221 of the connecting bridge 220. A thread 437 is arranged on the outer circumferential surface of the connection head 436, which engages with the fixing member 240.

The medical fixation device 400 according to yet another embodiment of the present invention has the coupler 430 coupled with the pair of guides 216 by rotating inside the slot 213, so the coupler 430 can be separated from the fixation bar 210 simply by rotating it inside the slot 213.

Also, as a modified embodiment of the medical fixation device shown in FIGS. 16 to 19, an embodiment where the coupler can rotate inside the slot but cannot move along the slot is also possible, although not shown in the drawings.

The exemplary embodiments of the present disclosure may also be described as follows.

A medical fixation device according to an embodiment of the present invention includes: a fixation bar including a slot and a guide; a connecting bridge disposed to intersect with the fixation bar; and a coupler having a base coupled to the guide such that at least a portion thereof is received in the slot and having a connection head protruding from the base to couple with the connecting bridge.

The slot may include: an entrance having a size through which the base can pass; and an extension part extending from the entrance in the longitudinal direction of the fixation bar, and the base may be coupled to the guide in the extension part so that movement in the thickness direction of the fixation bar is restricted.

The guide may be disposed in the extension part, and an inclined surface may be disposed at the end of the guide adjacent to the entrance.

The base may include: a base bottom in contact with one surface of the guide; a base top in contact with the other surface of the guide; and a groove disposed between the base bottom and the base top for the guide to be inserted.

The guide may be disposed in the slot as a pair facing each other.

The base may be capable of angle adjustment between the pair of guides to be coupled with or separated from the pair of guides.

At least one of the base bottom and the base top may have a width that can pass through a gap between the pair of guides.

One of the base bottom and the base top may have a width that can pass through a gap between the pair of guides, and the other has a width that cannot pass through the gap.

The connecting bridge may include a through hole into which the connection head is inserted.

The medical fixation device according to an embodiment of the present invention may further include: a fixing member coupled to the connection head to press the connecting bridge toward the base.

A medical fixation device according to another embodiment of the present invention includes: a fixation assembly including a guide portion; a connecting bridge attachable to the fixation assembly; and a coupler connected to the connecting bridge and capable of being coupled to or separated from the fixation assembly according to an engagement or disengagement angle relative to the guide portion.

The fixation assembly may comprise a plurality of bar segments, with at least one bar segment having the guide portion.

The connecting bridge may provide attachment support between the plurality of bar segments.

The coupler may comprise a screw and nut pair that permits manual attachment to and detachment from the fixation assembly.

The engagement or disengagement angle may be created by twisting or manipulating a spatial orientation of the coupler having at least a portion thereof received in the guide portion.

A device according to yet another embodiment of the present invention includes: a bar assembly insertable into a human body and having a curved shape in one direction to accommodate a medical fixation procedure related to a specific part of the human body; and a fastening member attachable to and detachable from at least one end of the bar assembly, wherein the bar assembly includes: a slot allowing positioning, attachment, and detachment of the fastening member; and a guide to accommodate the positioning of the fastening member and preventing the fastening member from deviating from the bar assembly during the positioning of the fastening member or when the fastening member is adjusted into place at a desired position after the positioning.

The slot may have a particular configuration to accommodate patient-tailored adjustments to the bar assembly and/or the fastening member in consideration of particular physical characteristics of a patient undergoing the medical fixation procedure.

The guide may have a particular configuration to accommodate patient-tailored adjustments to the bar assembly and/or the fastening member in consideration of particular physical characteristics of the patient undergoing the medical fixation procedure.

The fastening member may comprise a screw and nut pair that allows for manual attachment to and detachment from the bar assembly.

The bar assembly may comprise a plurality of bar members that are configured to cooperate with each other for the medical fixation procedure.

The above describes preferred examples of the present invention, but the scope of the present invention is not limited to the forms described and illustrated above.

For example, while the medical fixation device according to the present invention was described as being used for pectus excavatum correction surgery, the medical fixation device according to the present invention can be used in various medical fields such as orthopedic surgery or thoracic surgery.

Also, the shape of the fixation bar or connecting bridge can be variously changed according to the body tissue to be fixed or the surgical method.

Also, although the drawings show the connection head of the coupler in the form of a bolt and the fixing member in the form of a nut structure, the connection head and the fixing member can be configured to be coupled in other engagement methods, rotation methods, press-fit methods, etc.

The embodiments of this specification have been described in more detail with reference to the attached drawings, but this specification is not necessarily limited to such embodiments and can be variously modified and implemented within a range that does not depart from the technical idea of this specification. Therefore, the embodiments disclosed in this specification are not intended to limit the technical idea of this specification but to explain it, and the scope of the technical idea of this specification is not limited by these embodiments. Therefore, it should be understood that the embodiments described above are exemplary in all aspects and not restrictive.

## Claims

1. A medical fixation device (100) comprising:
a fixation bar (110) comprising a slot (113) and a guide (116);
a connecting bridge (120) disposed to intersect with the fixation bar (110); and
a coupler (130) having a base (131) coupled to the guide (116) such that at least a portion thereof is received in the slot (113) and having a connection head (136) protruding from the base (131) to couple with the connecting bridge (120).

2. The medical fixation device (100) of claim 1, wherein the slot includes:
an entrance (152) having a size through which the base can pass; and
an extension part (153) extending from the entrance in the longitudinal direction of the fixation bar, and
wherein the base is coupled to the guide in the extension part so that movement in the thickness direction of the fixation bar is restricted.

3. The medical fixation device of claim 2, wherein the guide is disposed in the extension part, and
wherein an inclined surface (217) is disposed at the end of the guide adjacent to the entrance.

4. The medical fixation device of any preceding claim, wherein the base includes:
a base bottom (232) in contact with one surface of the guide;
a base top (233) in contact with the other surface of the guide; and
a groove (235) disposed between the base bottom and the base top for the guide to be inserted.

5. The medical fixation device of any preceding claim, wherein the guide is disposed in the slot as a pair of guides facing each other.

6. The medical fixation device of claim 5, wherein the base is capable of angle adjustment between the pair of guides to be coupled with or separated from the pair of guides.

7. The medical fixation device of claim 6, wherein at least one of the base bottom and the base top has a width that can pass through a gap between the pair of guides.

8. The medical fixation device of claim 6, wherein one of the base bottom and the base top has a width that can pass through a gap between the pair of guides, and the other has a width that cannot pass through the gap.

9. The medical fixation device of any preceding claim, wherein the connecting bridge includes a through hole (221) into which the connection head is inserted.

10. The medical fixation device of any preceding claim, further comprising:
a fixing member (240) coupled to the connection head to press the connecting bridge toward the base.

11. The medical fixation device of claim 10, wherein the connection head has a thread (237) on an outer circumferential surface thereof, and the fixing member has an insertion hole (141) with a thread that engages with the thread (237) of the connection head.

12. The medical fixation device of any preceding claim, wherein the fixation bar has a curved shape so as to be insertable into a human body and to fix a specific part of the human body.

13. The medical fixation device of any preceding claim, wherein the fixation bar, the connecting bridge, and the coupler are each made of a biocompatible material.
